# EUROPEAN PATENT APPLICATION

(11) **EP 4 613 305 A1**
(43) Date of publication of application: **10.09.2025**
(21) Application number: 23886139.7
(22) Date of filing: 26.10.2023
(51) Int. Cl.: A61M 15/00

(54) **INHALER**

(30) Priority: 02.11.2022 KR 20220144244
(71) Applicant: KT&G Corporation, Daedeok-gu Daejeon 34337 (KR)
(72) Inventor: KIM, Moonwon, Daejeon 34128 (KR); JUNG, Yongmi, Daejeon 34128 (KR); SHIN, Jun Won, Daejeon 34128 (KR)
(74) Representative: Ter Meer Steinmeister & Partner
(86) International application number: PCT/KR2023/016784
(87) International publication number: WO 2024/096443

(57) **Abstract**

An inhaler according to an embodiment comprises: a main body that has a first surface and a second surface facing the first surface and forming an air inlet and has, on the inside, a longitudinal channel going from the first surface toward the second surface; a cartridge that is coupled to the first surface of the main body and in communication with the channel and contains an inhalable composition; and a rotating body disposed in the channel so as to be adjacent to the air inlet, wherein the rotating body can rotate to open or close the air inlet.

## Description

### TECHNICAL FIELD

An inhaler is disclosed.

### BACKGROUND ART

In general, an inhaler is a device used to inhale a composition such as a drug or other substance as a liquid or gas through an oral cavity or nasal cavity during an inhalation process. Such an inhaler is equipped with a container that accommodates an inhalable composition, and the composition may be sprayed from the container through a tube connected to the container and finally into the oral cavity or nasal cavity of a user through an intake.

In addition to a liquid inhaler and a vapor inhaler, such an inhaler includes a powder inhaler that sprays fine powder of the composition as microparticles and an aerosol inhaler that supplies the composition by aerosolizing the composition.

Recently, the application fields of dry powder inhalers have expanded, and the scope has expanded to not only disposable or multi-use powder inhalers, but also powder inhalers that inhale medicinal substances, functional substances, nicotine, and other preference products.

The above description is information the inventor(s) acquired during the course of conceiving the present disclosure or already possessed at the time and is not necessarily art publicly known before the present application was filed.

Prior art document: Korea Patent Publication No. 10-1759972

### DISCLOSURE OF THE INVENTION

### TECHNICAL GOALS

Embodiments are to provide an inhaler that may prevent foreign substances from entering from the outside or an inhalable composition from leaking out when the inhaler is not in use.

The technical goals obtainable from the embodiments are not limited to the above-mentioned technical goals, and other unmentioned technical goals may be clearly understood from the following description by one of ordinary skill in the art to which the present disclosure pertains.

### TECHNICAL SOLUTIONS

An inhaler according to an embodiment for achieving the goals includes a main body that includes a first surface and a second surface facing the first surface and having an air inlet formed thereon and has a channel formed inside in a longitudinal direction from the first surface toward the second surface, a cartridge coupled to the first surface of the main body, communicating with the channel, and containing an inhalable composition, and a rotating body disposed in the channel so as to be adjacent to the air inlet, wherein the rotating body may be rotatable to open or close the air inlet.

According to an aspect, the rotating body may include a rotating cylinder inserted into the channel, a rotation opening formed at one end of the rotating cylinder, and a protruding member formed at one side of the rotating cylinder, wherein, when the positions of the rotating opening and the air inlet are aligned, the cartridge may be communicated with the air inlet.

According to an aspect, the main body may be formed at a position adjacent to the first surface, a slit may be formed through which the protruding member penetrates, and the slit may guide a movement path of the protruding member.

According to an aspect, the slit may include a first slit extending in a transverse direction crossing the longitudinal direction, and the first slit may guide a rotation path of the protruding member.

According to an aspect, the slit may include a second slit formed in a "flattened U" shape extending in the longitudinal direction and a transverse direction crossing the longitudinal direction, and the second slit may guide a rotational and vertical movement path of the protruding member.

According to an aspect, an inner side surface of the rotating cylinder may be formed in a spiral shape.

According to an aspect, the rotation opening may have a same shape as the air inlet.

### EFFECTS OF THE INVENTION

An inhaler according to an embodiment has an effect of preventing foreign substances from entering from the outside or an inhalable composition from leaking out when the inhaler is not in use.

The effects of the inhaler according to an embodiment are not limited to the above-mentioned effects, and other unmentioned effects can be clearly understood from the following description by one of ordinary skill in the art to which the present disclosure pertains.

### BRIEF DESCRIPTION OF DRAWINGS

FIG. 1 schematically illustrates an inhaler according to an embodiment.
FIG. 2 illustrates a rotating body of an inhaler according to an embodiment.
FIG. 3 illustrates a main body of an inhaler according to an embodiment.
FIG. 4 illustrates a main body having a slit in a different type.

The accompanying drawings illustrate preferred embodiments of the present invention and are provided together with the detailed description for better understanding of the technical idea of the present invention. Therefore, the present invention should not be construed as being limited to the embodiments set forth in the drawings.

### BEST MODE FOR CARRYING OUT THE INVENTION

Hereinafter, embodiments are described in detail with reference to the illustrative drawings. Regarding the reference numerals assigned to the components in the drawings, it should be noted that the same components are designated by the same reference numerals, wherever possible, even though they are shown in different drawings. Furthermore, in the following description of the present embodiments, a detailed description of publicly known configurations or functions incorporated herein will be omitted when it is determined that the detailed description obscures the subject matters of the present embodiments.

In addition, the terms first, second, A, B, A, and B may be used to describe components of the embodiments. These terms are used only for the purpose of discriminating one component from another component, and the nature, the sequences, or the orders of the components are not limited by the terms. When one component is described as being "connected", "coupled", or "attached" to another component, it should be understood that one component may be connected or attached directly to the other component, and an intervening component may also be "connected", "coupled", or "attached" to the components.

The same name may be used to describe an element included in the embodiments described above and an element having a common function. Unless otherwise mentioned, the descriptions of the embodiments may be applicable to the following embodiments and thus, duplicated descriptions will be omitted for conciseness.

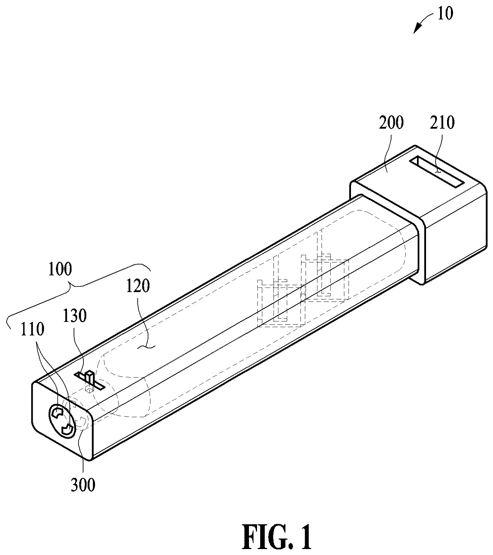

FIG. 1 schematically illustrates an inhaler 10 according to an embodiment.

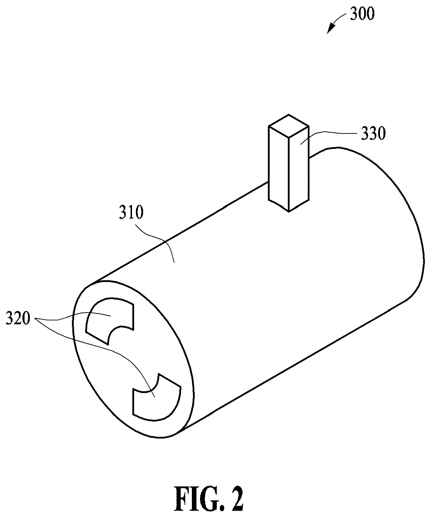

FIG. 2 illustrates a rotating body 300 of the inhaler 10 according to an embodiment.

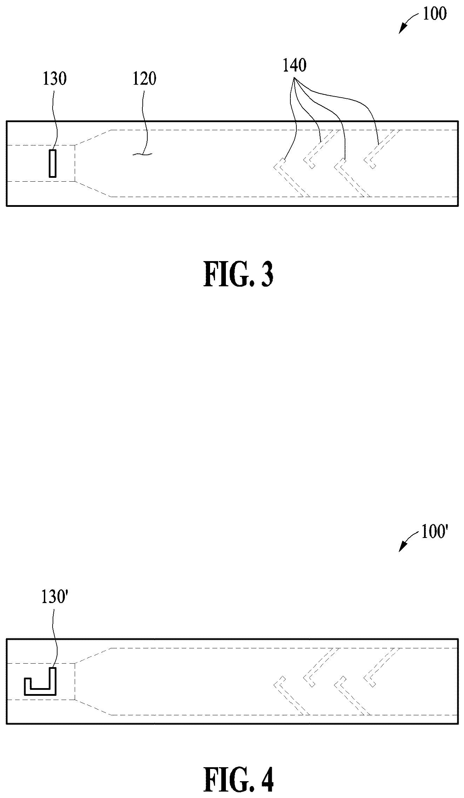

FIG. 3 illustrates a main body 100 of the inhaler 10 according to an embodiment.

FIG. 4 illustrates a main body 100' having a slit 130' in a different type.

Referring to FIG. 1, the inhaler 10 according to an embodiment may include the main body 100, a cartridge 200, and the rotating body 300.

The main body 100 may include a first surface, a second surface facing the first surface, and a plurality of side surfaces surrounding the first surface and the second surface. The cartridge 200 may be coupled to the first surface. An air inlet 110 may be formed on the second surface. A channel 120 penetrating the first surface and the second surface may be formed inside the main body 100. The channel 120 may be a passage through which an inhalable composition for transfer to a user is transferred. Here, the inhalable composition may be transferred in a longitudinal direction from the first surface to the second surface.

In addition, the main body 100 may be provided with a flow distribution structure for controlling the degree of inhalation of the inhalable composition at an end portion adj acent to the second surface.

Furthermore, the main body 100 may include materials such as polylactic acid (PLA), acrylonitrile butadiene styrene copolymer (ABS), polycarbonate (PC), polypropylene (PP), etc. and may also include other biodegradable (eco-friendly) materials or metal.

The cartridge 200 may be coupled to the first surface of the main body 100. The cartridge 200 may contain the inhalable composition inside and may be connected internally with the channel of the main body 100. The cartridge 200 may contain one or multiple doses of the inhalable composition. The inhalable composition may include substances such as nicotine, nicotine salts, caffeine, taurine, vitamins, etc. In addition, the inhalable composition may include flavored particles such as natural flavor particles, menthol, etc. The inhalable composition may be provided in the form of, for example, dry powder. Here, the dry powder used in the inhaler 10 may have or not have a carrier.

As described above, the inhalable composition may be transferred through the channel 120, and thus, the user may inhale the inhalable composition into the body.

In addition, the cartridge 200 may be detachably coupled to the first surface. Therefore, when the inhalable composition contained in the cartridge 200 is exhausted, a new cartridge 200 may be used as a replacement and mounted on the main body 100. Here, the cartridge 200 may be fastened by magnets or in a fitting manner

A mesh net may be provided between the main body 100 and the cartridge 200. The mesh net may include the same material as a material of the main body 100, such as PLA, for example. In addition, the mesh net may include a chemically stable metal with no inhalation toxicity, such as stainless steel, for example.

Furthermore, the inhaler 10 according to an embodiment may be a device that transfers the inhalable composition including dry powder and the like by utilizing an inertial force of particles of the inhalable composition, and when the particles of the transferred inhalable composition are larger than a certain size, the inhaler 10 may cause the user to cough. Accordingly, a plurality of baffles 140 may be provided in a zigzag structure as a filter structure to prevent inflow of particles of an unspecified size.

In addition, in the case of a dry powder inhaler 10, there may be an advantage of being easy to carry, but due to a method of directly inhaling fine powder, contamination of the inside of the main body 100 may cause negative consequences for a consumer. Therefore, the inhaler 10 according to an embodiment may be provided with a structure that may open and close the inside of the main body 100, thereby preventing contamination of the inhaler 10 that is not in use and at the same time preventing residual powder from being discharged to the outside.

The rotating body 300 may be disposed inside the channel 120 so as to be adjacent to the air inlet 110. The rotating body 300 may be rotated to open or close the air inlet 110 inside the channel 120 or to adjust the degree of inhalation of the inhalable composition.

Referring to FIG. 2, the rotating body 300 may include a rotating cylinder 310, a rotation opening 320, and a protruding member 330.

The rotating cylinder 310 may be inserted into the channel 120. The rotating cylinder 310 may be formed in a cylindrical shape, for example. Both end portions of the rotating cylinder 310 may be formed in an open shape. Therefore, even if the rotating cylinder 310 is inserted into the channel 120, the channel 120 may be communicated with the outside through the air inlet 110.

An inner surface of the rotating cylinder 310 may be formed in a vortex shape having a spiral shape. Accordingly, a vortex may be generated in the flow of the inhalable composition.

The rotation opening 320 may be formed at one end of the rotating cylinder 310. The rotating cylinder 310 may be disposed so that the rotation opening 320 and the air inlet 110 of the main body 100 are adjacent to each other.

The rotation opening 320 may be formed in a fan shape or a dot shape, for example. The rotation opening 320 may be formed in the same shape as the air inlet 110. Therefore, when positions of the rotation opening 320 and the air inlet 110 are aligned, the cartridge 200 may be communicated with the air inlet 110, and the channel 120 may be opened. In addition, when the positions of the rotation opening 320 and the air inlet 110 are not aligned, the cartridge 200 may be communicated with the channel 120 but may not be communicated with the air inlet 110. That is, the channel 120 may be closed by the rotating body 300.

The protruding member 330 may be formed on one side of the rotating cylinder 310. The protruding member 330 may protrude in a radial direction from a side surface of the rotating cylinder 310.

Referring again to FIG. 1, the main body 100 may further include a slit 130.

The slit 130 may be formed at a position adjacent to the first surface, and the protruding member 330 may penetrate through the slit 130. The protruding member 330 may move along the slit 130, and the rotating cylinder 310 may rotate or move vertically in the longitudinal direction according to the movement of the protruding member 330. That is, the slit 130 may guide a movement path of the protruding member 330.

FIG. 3 and FIG. 4 illustrate main bodies 100 and 100' having various shapes of slits 130 and 130'.

The main body 100 of FIG. 3 may include a first slit 130, and the main body 100' of FIG. 4 may include a second slit 130'.

Referring to FIG. 3, the first slit 130 of the main body 100 according to an embodiment may be formed in a shape extending in a transverse direction crossing the longitudinal direction. Here, the protruding member 330 may rotate in a circumferential direction of the rotating cylinder 310 along the first slit 130. Therefore, the first slit 130 may guide a rotation path of the protruding member 330.

Referring to FIG. 4, the second slit 130' of the main body 100' according to another embodiment may be formed in a flattened "U" shape that extends continuously in the longitudinal direction and the transverse direction crossing the longitudinal direction. Here, the protruding member 330 may rotate in the circumferential direction of the rotating cylinder 310 along the second slit 130' or may move vertically in the longitudinal direction of the main body 100'. Therefore, the second slit 130' may guide a rotation and vertical movement path of the protruding member 330.

As described above, the inhaler 10 according to an embodiment may be provided with the rotating body 300 having the rotation opening 320 corresponding in size or shape to the air inlet 110 of the main body 100, and the protruding member 330 formed on the rotating body 300 may be combined to form the slit 130, 130', which limits a range of movement of the protruding member 330, on one side of the main body 100, 100', so that the air inlet 110 communicating with the channel 120 may be selectively opened and closed through vertical or rotational movement of the rotating body 300, and the degree of opening of the air inlet 110 may be easily adjusted.

While the embodiments of the present disclosure have been described above with reference to specific components, and limited embodiments and drawings, the above descriptions are merely for better understanding of the present disclosure, and it will be apparent to one of ordinary skill in the art that various changes in form and details may be made in these embodiments without departing from the spirit and scope of the claims and their equivalents. For example, suitable results may be achieved if the described techniques are performed in a different order and/or if components in a described system, architecture, device, or circuit are combined in a different manner and/or replaced or supplemented by other components or their equivalents. Accordingly, the scope of the present disclosure is defined not by the detailed description, but by the claims and their equivalents, and all variations within the scope of the claims and their equivalents are to be construed as being included in the disclosure.

## Claims

1. An inhaler comprising:
a main body that comprises a first surface and a second surface facing the first surface and having an air inlet formed thereon and has a channel formed inside in a longitudinal direction from the first surface toward the second surface;
a cartridge coupled to the first surface of the main body, communicating with the channel, and containing an inhalable composition; and
a rotating body disposed in the channel so as to be adjacent to the air inlet,
wherein the rotating body is rotatable to open or close the air inlet.

2. The inhaler of claim 1, wherein the rotating body comprises:
a rotating cylinder inserted into the channel;
a rotation opening formed at one end of the rotating cylinder; and
a protruding member formed at one side of the rotating cylinder,
wherein, when the positions of the rotating opening and the air inlet are aligned, the cartridge is communicated with the air inlet.

3. The inhaler of claim 2, wherein the main body is formed at a position adjacent to the first surface, a slit is formed through which the protruding member penetrates, and
the slit guides a movement path of the protruding member.

4. The inhaler of claim 3, wherein the slit comprises a first slit extending in a transverse direction crossing the longitudinal direction, and
the first slit guides a rotation path of the protruding member.

5. The inhaler of claim 3, wherein the slit comprises a second slit formed in a "flattened U" shape extending in the longitudinal direction and a transverse direction crossing the longitudinal direction, and
the second slit guides a rotational and vertical movement path of the protruding member.

6. The inhaler of claim 2, wherein an inner side surface of the rotating cylinder is formed in a spiral shape.

7. The inhaler of claim 2, wherein the rotation opening has a same shape as the air inlet.
